# EUROPEAN PATENT APPLICATION

(11) **EP 4 850 811 A1**
(43) Date of publication of application: **09.12.2026**
(21) Application number: 25814666.1
(22) Date of filing: 15.05.2025
(51) Int. Cl.: B01J 35/00, B01J 23/10, C07C 2/84, C07C 11/04, B01J 37/18

(54) **SINGLE-ATOM CATALYST, AND PREPARATION METHOD THEREFOR AND USE THEREOF**

(30) Priority: 28.05.2024 CN 202410675724
(71) Applicant: China Petroleum & Chemical Corporation, Beijing 100728 (CN); Sinopec (Beijing) Research Institute of Chemical Industry Co., Ltd., Beijing 100013 (CN)
(72) Inventor: WU, Jiehua, Beijing 100013 (CN); XUE, Wei, Beijing 100013 (CN); WANG, Xue, Beijing 100013 (CN); BAI, Jie, Beijing 100013 (CN); ZHANG, Mingsen, Beijing 100013 (CN)
(74) Representative: karo IP
(86) International application number: PCT/CN2025/094993
(87) International publication number: WO 2025/246948

(57) **Abstract**

The present invention relates to the technical field of oxidative coupling of methane, and discloses a single-atom catalyst comprising a carrier and an active metal supported on the carrier, a preparation method therefor, and a use thereof. In the single-atom catalyst provided by the present invention, the active metal forms single-atom active sites on the carrier, utilizing the synergism between the active metal and the carrier, and between the active metals, to obtain a better catalytic effect for the oxidative coupling reaction of methane.

## Description

### Technical Field

The present invention relates to the technical field of catalysts, and specifically, to a single-atom catalyst, a preparation method therefor, and a use thereof in the oxidative coupling of methane.

### Background Art

The high temperature of the oxidative coupling reaction of methane leads to a rapid deactivation of the catalyst, which is one of the reasons why the current oxidative coupling technology of methane is difficult to be industrialized. Although researchers have made improvements in various aspects, the reaction effect is still unsatisfactory.

A single-atom catalyst refers to a catalyst where active metals are supported on the surface of a carrier in the form of individual atoms, and primarily bonded to the carrier surface through the heteroatomic bonding. A single-atom catalyst does not mean that individual zero-valent metal atoms are the active centers; the single atoms also undergo coordination interactions, such as electron transfer, with other atoms of the carrier, often exhibiting a certain charge property. Single-atom catalysts have been widely and deeply studied due to their extremely high atomic utilization rate and excellent catalytic performance, and have become a hot topic in the field of catalysis in recent years. Single-atom catalysts have also shown good performance in the oxidative coupling reaction of methane. However, conventional single-atom catalysts in the prior art only contain one type of metal center, the number of atoms in the single-atom state is insufficient, and the distance between active metal atoms is too large, making it difficult to break the linear relationships present in many catalytic processes. Their performance still needs a significant improvement. Additionally, most single-atom catalysts in the prior art have the problems, such as the low metal loading and the relatively weak interaction between individual atoms and the carrier material, which largely limits the practical application of the single-atom catalysts in the field of catalysis.

### Summary of the Invention

The object of the present invention is to overcome the problems of the prior art, such as the need for improvement in the catalytic performance of single-atom catalysts used for the oxidative coupling of methane, and to provide a single-atom catalyst containing at least one active metal, a preparation method therefor and a use thereof. Compared with the single-atom catalysts in the prior art, the single-atom catalyst of the present invention effectively improves the catalytic activity of the active metals in the catalyst, can significantly reduce the reaction temperature of the oxidative coupling of methane, making the industrial production of the oxidative coupling of methane possible.

In particular, the single-atom catalyst of the present invention contains two active metals as active components, which have a synergism and form active sites of the two active metals on the carrier.

In a first aspect, the present invention provides a single-atom catalyst, which comprises a carrier and an active metal supported on the carrier, wherein the carrier is an oxide carrier, the active metal is selected from at least one of Group IA metals, Group IIA metals, Group IIB metals, Group IIIB metals, Group VIB metals and Group VIIB metals,
wherein the active metal present in a single-atom state on the carrier accounts for ≥70% of the total amount of the active metal.

A second aspect of the present invention provides a method for preparing a single-atom catalyst, comprising: Step 1: preparing a mixed solution from a precursor of at least one metal, a nitrogen-containing compound ligand containing two or more nitrogen atoms, and a solvent, and contacting the mixed solution with a carrier optionally subjected to a reduction treatment, to obtain a mixture; Step 2: subjecting the mixture obtained in Step 1 to an ultrasonic dispersion, followed by filtration and drying, to obtain a catalyst precursor; Step 3: drying and calcining the catalyst precursor obtained in Step 2, and cooling to obtain a single-atom catalyst;
wherein the carrier is an oxide carrier, and the metal is selected from at least one of Group IA metals, Group IIA metals, Group IIB metals, Group IIIB metals, Group VIB metals and Group VIIB metals.

A third aspect of the present invention provides a single-atom catalyst prepared by the method according to the second aspect.

A fourth aspect of the present invention provides a use of the catalyst according to the first or third aspect or the method according to the second aspect in an oxidative coupling reaction of methane.

A fifth aspect of the present invention provides a method for producing C²⁺ hydrocarbons(i.e. hydrocarbons having two or more carbon atoms), which comprises contacting a feed gas comprising CH₄ and O₂ with the catalyst according to the first or third aspect to carry out an oxidative coupling reaction of methane.

### Description of Drawings

Figures 1-A and 1-B are XAFS spectra (X-ray absorption fine structure spectra) showing the existence of metal sites Ba and Sr of the present invention in a single-atom state.
Figures 2-A to 2-F are the k. r. q space XAFS fitting spectra of the single-atom catalyst CAT-A prepared in Example 1.
Figures 3-A to 3-F are the k. r. q space XAFS fitting spectra of the single-atom catalyst CAT-B prepared in Example 2.

### Mode of Carrying Out the Invention

In a first aspect, the present invention provides a single-atom catalyst, which comprises a carrier and an active metal supported on the carrier, wherein the carrier is an oxide carrier, the active metal is selected from at least one of Group IA metals, Group IIA metals, Group IIB metals, Group IIIB metals, Group VIB metals, and Group VIIB metals,
wherein the active metal present in a single-atom state on the carrier accounts for ≥70%, preferably ≥80%, more preferably ≥90% of the total amount of the active metal.

In the present invention, the active metal exists in a single-atom state, but this does not mean that the active metal cannot exist in other forms.

The existence form of the active metal in the single-atom catalyst can be characterized by synchrotron radiation methods (such as XAFS, etc.), and the test results are fitted for determination.

The proportion of the active metal in a single-atom state relative to the total amount of the active metal is determined as follows: characterization is performed using a synchrotron radiation method; ten random positions of the sample to be tested are selected for testing, and the fitting results indicate the ratio of the number of the positions in a single-atom state to the total number of the positions. For example, if nine out of ten to-be-tested positions on the same sample show a single-atom form, it is considered that the active metal in a single-atom state accounts for 90% of the total amount of the active metal. Here, ten positions are only used to explain the calculation method, and in actual testing, more than ten positions can be used in practice.

In some embodiments, in the single-atom catalyst of the present invention, based on a non-oxygen element in the carrier and a metal element in the active metal, the molar ratio of the carrier (hereinafter also denoted as S) to the active metal is 2-500:1, preferably 4-500:1, more preferably 4-400:1. "Molar ratio of the carrier to the active metal" refers to the molar ratio of the non-oxygen element in the oxide serving as the carrier to the total loading amount of metal elements in the active metal supported on the carrier.

In some embodiments, in the single-atom catalyst of the present invention, the molar ratio of the carrier S to the active metal can be 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 30:1, 40:1, 50:1, 80:1, 100:1, 120:1, 150:1, 180:1, 200:1, 220:1, 250:1, 280:1, 300:1, 320:1, 350:1, 280:1, 400:1, 420:1, 450:1, 480:1, 500:1, or can be a range formed by any two of the above ratios or any intermediate value within the range.

In some embodiments, in the single-atom catalyst of the present invention, there is a situation that the spacing between the active metal atoms is 3.8-4.0 Å, for example, about 3.9 Å. Preferably, the proportion of the single-atom-state metals having a spacing of 3.8-4.0 Å between the active metal atoms relative to the total single-atom-state active metals is ≥25%, for example, 28% and 40%.

In some embodiments, an M1-(O)ₙ-M2 structural form is present in the single-atom catalyst of the present invention, wherein M1 and M2 respectively represent the same active metal or two different active metals, n represents 1 or 2, preferably 1, and the spacing between M1 and M2 is 3.8-4.0 Å, for example, about 3.9 Å. Preferably, the proportion of M1 and M2 in a single-atom state having a spacing of 3.8-4.0 Å between M1 and M2 relative to the total single-atom-state active metals is ≥25%, for example, 28% and 40%.

In some embodiments, the carrier is selected from at least one of lanthanum oxide, aluminum oxide, cerium oxide and silicon oxide, preferably lanthanum oxide.

Preferably, the carrier is in a crystalline or amorphous state.

Preferably, the specific surface area of the carrier may be 1-500 m²/g, more preferably 20-400 m²/g.

Preferably, the active metal is selected from at least one of Sr, Ba, Mn, Mg, Ce, Ca, Li, Na, K, Zn and W, preferably a combination of any two, particularly preferably Sr and Ba.

In the single-atom catalyst of the present invention, when at least two active metals, preferably two active metals, are present, the active metals can be a combination of at least two metals selected from the same Group, or a combination of at least two metals selected from different Groups.

Preferably, the active metal can be Ba and/or Sr.

Preferably, the active metal can be any one of the following combinations: Sr and Ba, Sr and Mn, Ba and Mg, Ce and Mg, Ba and Li, Ca and K, Ba and Mn, Zn and W, or Mn and Na, particularly preferably Sr and Ba.

In particular, when two active metals are present in the single-atom catalyst of the present invention, a single-atom catalyst containing the two active metals is formed, wherein the two active metals, for example, form an M1-(O)ₙ-M2 structural form, where M1 and M2 respectively represent two different active metals, n represents 1 or 2, preferably 1, and the spacing between M1 and M2 is preferably 3.8-4.0 Å, for example, about 3.9 Å.

In the single-atom catalyst containing two active metals, two different active metals simultaneously exist in a single-atom state and have a certain interaction (e.g., synergistic effect, geometric effect, and electronic effect), for example, with a spacing of 3.8-4.0 Å between them. The interaction between the two active metals is not merely a simple sum of the functions of each individual metal atom, but also this interaction helps to regulate the electronic structure of the catalytically active sites, effectively improving the catalytic performance, and thereby enabling the better catalytic activity and effect in the oxidative coupling reaction of methane.

In the case of a single-atom catalyst containing two active metals, the active metal is preferably selected from a combination of any two from Sr, Ba, Mn, Mg, Ce, Ca, Li, Na, K, Zn and W, particularly preferably Sr and Ba.

In the case of a single-atom catalyst containing two active metals, the active metals can be a combination of two metals from the same Group, or a combination of two metals from different Groups.

In the case of a single-atom catalyst containing two active metals, the active metal may preferably be any one of the following combinations: Sr and Ba, Sr and Mn, Ba and Mg, Ce and Mg, Ba and Li, Ca and K, Ba and Mn, Zn and W, or Mn and Na, particularly preferably Sr and Ba.

In the case of a single-atom catalyst containing two active metals, the single-atom catalyst of the present invention can be denoted as M1M2/S, where M1 and M2 respectively represent the active metals, and S represents the carrier.

According to some preferred embodiments of the present invention, based on the non-oxygen element in the carrier and the metal elements in the active metals, the molar ratio of the carrier S to the active metals M1M2 (i.e., the total amount of active components) is 2-500:1, preferably 4-500:1, more preferably 4-400:1. "Molar ratio of the carrier to the active metals" refers to the molar ratio of the non-oxygen element in the oxide serving as the carrier to the total loading amount of the metal elements of the two different active metals supported on the carrier. For example, in a catalyst using lanthanum oxide as the carrier and Sr and Ba as active metals, the "molar ratio of the carrier to the active metals" is the ratio of the molar amount of La in the carrier to the total molar amount of Sr and Ba supported on the carrier; for another example, in a catalyst using silicon oxide as the carrier and Sr and Ba as active metals, the "molar ratio of the carrier to the active metals" is the ratio of the molar amount of Si in the carrier to the total molar amount of Sr and Ba supported on the carrier.

In the case of a single-atom catalyst containing two active metals, the molar ratio of the carrier S to the active components M1 and M2 can be 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 30:1, 40:1, 50:1, 80:1, 100:1, 120:1, 150:1, 180:1, 200:1, 220:1, 250:1, 280:1, 300:1, 320:1, 350:1, 280:1, 400:1, 420:1, 450:1, 480:1, 500:1, or can also be a range formed by any two above ratios or any intermediate value within the range.

In the case of a single-atom catalyst containing two active metals, the present invention has no particular limitation on the content ratio of the two active metals in the single-atom catalyst. However, to achieve better catalytic performance, according to some preferred embodiments of the present invention, in the single-atom catalyst M1M2/S containing two active metals, based on metal elements, the molar ratio of M1 to M2 can be 1:0.1-10, preferably 1:0.1-5. For example, it can be 1:0.1, 1:1, 1:1.5, 1:2, 1:2.5, 1:3, 1:3.5, 1:4, 1:4.5, 1:5, or can also be any intermediate ratio within a ratio range formed by any two above ratios.

A second aspect of the present invention provides a method for preparing a single-atom catalyst, comprising:
Step 1: preparing a mixed solution from a precursor of at least one metal, a nitrogen-containing compound ligand containing two or more nitrogen atoms, and a solvent, and contacting the mixed solution with a carrier optionally subjected to a reduction treatment, to obtain a mixture;
Step 2: subjecting the mixture obtained in Step 1 to an ultrasonic dispersion, followed by filtration and drying, to obtain a catalyst precursor;
Step 3: drying and calcining the catalyst precursor obtained in Step 2, and cooling to obtain a single-atom catalyst;
wherein the carrier is an oxide carrier, and the metal is selected from at least one of Group IA metals, Group IIA metals, Group IIB metals, Group IIIB metals, Group VIB metals, and Group VIIB metals.

In some embodiments, the carrier is selected from at least one of lanthanum oxide, aluminum oxide, cerium oxide and silicon oxide, preferably lanthanum oxide.

In some embodiments, the metal is selected from at least one of Sr, Ba, Mn, Mg, Ce, Ca, Li, Na, K, Zn and W, preferably a combination of any two, particularly preferably Sr and Ba.

When at least two active metals are used, the metals can be a combination of at least two metals selected from the same Group, or a combination of at least two metals selected from different Groups.

Preferably, the metal can be Ba and/or Sr.

Preferably, the metal can be any one of the following combinations: Sr and Ba, Sr and Mn, Ba and Mg, Ce and Mg, Ba and Li, Ca and K, Ba and Mn, Zn and W, or Mn and Na, particularly preferably Sr and Ba.

Preferably, the precursor is a salt, which is preferably selected from water-soluble salts, preferably at least one of nitrates, sulfates and halides. Said halides are, for example, fluorides, chlorides and bromides.

In some embodiments, the nitrogen-containing compound ligand is selected from (organic) compounds containing an amine group.

In some embodiments, the nitrogen-containing compound ligand is selected from at least one of diethylenetriamine, ethylenediamine tetraacetic acid, bipyridine, porphyrin and phthalocyanine.

Preferably, the solvent is water or an organic solvent, more preferably, the solvent is at least one of water, alcohols having no more than 5 carbon atoms, and ketones having no more than 5 carbon atoms, even more preferably, the solvent is at least one of water, methanol, ethanol and acetone.

In some embodiments, in the mixed solution, the molar concentration ratio of the ligand to the precursor, calculated based on metal ions, is 2-5:1. For example, it can also be 2:1, 2.5:1, 3:1, 3.5:1, 4:1, 4.5:1, 5:1, or can be a range formed by any two above ratios or any intermediate value within the range.

In order to create defects, which facilitate the loading and the dispersion of the ligand and the metal atoms, on the surface of the carrier, the carrier is pretreated before contacting with the mixed solution according to a preferred embodiment of the present invention. Any method capable of creating the above-mentioned defects on the carrier surface is applicable to the present invention. According to some preferred embodiments of the present invention, the pretreatment method can be a heat treatment A performed on the carrier under a reducing atmosphere.

Preferably, the reducing atmosphere is provided by H₂ and/or CO.

Preferably, the conditions of the heat treatment A include: a temperature of 300-500°C, time of 0.5-5 h. For example, the carrier can be left to stand under the above conditions.

According to a preferred embodiment of the present invention, calculated based on metal ions, the amount of the carrier as used is such that the molar ratio of the carrier to a metal precursor is 2-500:1, preferably 4-500:1, more preferably 4-400:1. "Molar ratio of the carrier to a metal precursor" refers to the molar ratio of the non-oxygen element in the oxide serving as the carrier to the total amount of the metal ions generated by the metal precursor in the mixed solution. For example, with lanthanum oxide as the carrier and the water-soluble salts of Sr and Ba as the precursors, the molar ratio of the carrier to the metal precursor is the ratio of the molar amount of La provided by lanthanum oxide to the sum of the molar amount of Sr and the molar amount of Ba provided by the metal precursors.

In the method provided by the present invention, the amount of the carrier as used is such that the molar ratio of the carrier to the metal precursor can be 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 21:1, 22:1, 23:1, 24:1, 25:1, 30:1, 40:1, 50:1, 80:1, 100:1, 120:1, 150:1, 180:1, 200:1, 220:1, 250:1, 280:1, 300:1, 320:1, 350:1, 280:1, 400:1, 420:1, 450:1, 480:1, 500:1, or can be a range formed by any two above ratios or any intermediate value within the range.

In order to make the supported metal precursor distribution more uniform, according to a preferred embodiment of the present invention, in Step 2, after the ultrasonic treatment, a heat treatment B is further performed.

Preferably, the conditions of the ultrasonic treatment include: a temperature of 40-70°C, an ultrasonic frequency of 20-100 kHz, an ultrasonic power of 200-500 W, an ultrasonic time 0.5-5 h.

For example, the ultrasonic frequency can be 20 kHz, 30 kHz, 40 kHz, 50 kHz, 60 kHz, 70 kHz, 80 kHz, 90 kHz, 100 kHz, or can be a range formed by any two above values or any intermediate value within the range.

For example, the ultrasonic power can be 200 W, 250 W, 300 W, 350 W, 400 W, 450 W, 500 W, or can be a range formed by any two above values or any intermediate value within the range.

Preferably, stirring is performed during the ultrasonic treatment. The purpose of stirring is mainly to increase the mixing uniformity between the metal precursor and the carrier, and any stirring conditions capable of achieving this purpose are applicable to the present invention. For example, for a 500 mL treatment system, stirring can be performed at a stirring speed of 100-500 rpm.

Preferably, the conditions of the heat treatment B include: a temperature of 40-90°C, time of 1-10 h. Preferably, stirring is performed during the heat treatment B. The purpose of stirring is mainly to prevent an uneven distribution of active components caused by the surface solvent evaporation, and any stirring conditions capable of achieving this purpose are applicable to the present invention. For example, for a 500 mL treatment system, stirring can be performed at a stirring speed of 200-600 rpm.

According to a preferred embodiment of the present invention, the drying conditions are such that the solvent content in the carrier loaded with the metal precursor and the ligand does not exceed 1% by weight, preferably 0.1-0.7% by weight. "Solvent content" refers to the content of the residual solvent in the carrier loaded with the precursor and the ligand.

Preferably, the conditions of the drying include: a temperature of 70-120°C, time of 10-30 hours.

According to a preferred embodiment of the present invention, the calcination method includes: calcining at 100-650°C for 30-300 minutes.

Preferably, the calcination is carried out under a protective atmosphere, for example, provided by air and/or nitrogen.

A third aspect of the present invention provides the single-atom catalyst prepared by the method according to the second aspect.

A fourth aspect of the present invention provides a use of the single-atom catalyst according to the first or third aspect in an oxidative coupling reaction of methane, or a use of the method according to the second aspect in an oxidative coupling reaction of methane. "Use ... in an oxidative coupling reaction of methane" can be understood as using the single-atom catalyst of the present invention for (any scale of) oxidative coupling reaction of methane, which can also include: when using the oxidative coupling method of methane for the industrial production (e.g., for producing the hydrocarbons having two or more carbon atoms, etc.), the method of the present invention is used for preparing the single-atom catalyst and the prepared single-atom catalyst is used for the reaction, etc.

A fifth aspect of the present invention provides a method for producing the hydrocarbons having two or more carbon atoms, which comprises contacting a feed gas comprising CH₄ and O₂ with the single-atom catalyst according to the first or third aspect, to carry out an oxidative coupling reaction of methane.

According to a preferred embodiment of the present invention, the molar ratio of CH₄ to O₂ in the feed gas is 2-10:1, preferably 2-8:1.

For example, the molar ratio of CH₄ to O₂ in the feed gas can be 2:1, 3:1, 4:1, 5:1, 6:1, 7:1, 8:1, or can be a range formed by any two above ratios or any intermediate value within the range.

According to a preferred embodiment of the present invention, the conditions for the oxidative coupling reaction of methane include: a reaction temperature of not lower than 450°C, preferably 480-800°C; a feed gas space velocity, based on CH₄, of 20,000-200,000 mL/(g·h), preferably 30,000-180,000 mL/(g·h). mL/(g·h) refers to the amount of feed gas (mL) passed through a unit amount of catalyst (g) per unit time (h).

For example, the temperature of the oxidative coupling reaction of methane can be 450°C, 460°C, 470°C, 480°C, 490°C, 500°C, 510°C, 520°C, 530°C, 540°C, 550°C, 575°C, 600°C, 625°C, 650°C, 675°C, 700°C, 725°C, 750°C, or can be a range formed by any two above values or any intermediate value within the range.

For example, the space velocity of the feed gas based on CH₄ for the oxidative coupling reaction of methane can be 20,000 mL/(g·h), 30,000 mL/(g·h), 40,000 mL/(g·h), 50,000 mL/(g·h), 60,000 mL/(g·h), 70,000 mL/(g·h), 80,000 mL/(g·h), 90,000 mL/(g·h), 100,000 mL/(g·h), 110,000 mL/(g·h), 120,000 mL/(g·h), 130,000 mL/(g·h), 140,000 mL/(g·h), 150,000 mL/(g·h), 160,000 mL/(g·h), 180,000 mL/(g·h), 200,000 mL/(g·h), or can be a range formed by any two above values or any intermediate value within the range.

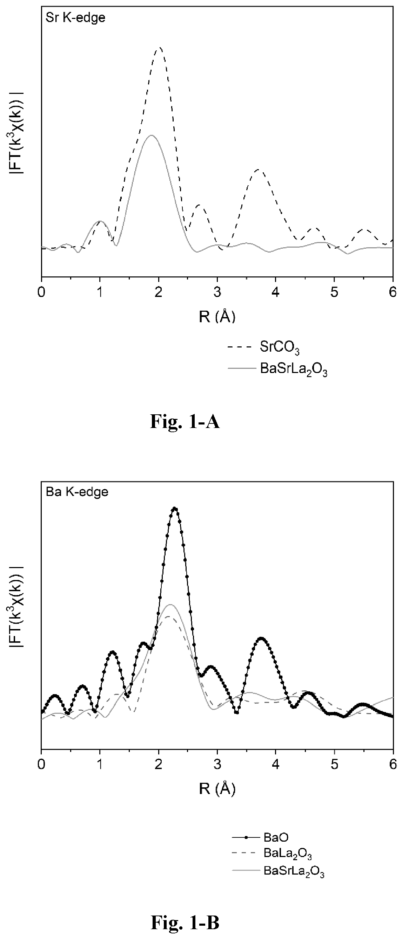

For the single-atom catalyst of the present invention, for example, the single-atom catalyst containing two active metals of barium and strontium (e.g., catalyst CAT-A of Example 1), the coordination environments of the barium atom and the strontium atom are characterized separately using XAFS (see Figures 1-A and 1-B). It can be seen that, compared with the standard sample of barium oxide, the main peak in the R space for the single-atom catalyst containing two active metals in the present invention is consistent with the Ba-O peak in barium oxide, proving that the coordination form of barium in this sample is present as a barium-oxygen bond; in the barium oxide sample, there is a barium- barium bond (with a peak between 3 and 4.5 in the figure), while there is no corresponding peak in the catalyst of the present invention, proving that there is no Ba-Ba bond and the coordination form of Ba is a Ba-O bond; likewise, it is proved that there is no Sr-Sr bond in the catalyst of the present invention, and the coordination form of Sr is an Sr-O bond. Based on the bond lengths and the coordination numbers, combined with the DFT fitting results, it is confirmed that the active metal atoms of Ba and Sr in the single-atom catalyst of the present invention exist in a single-atom state. In the structures obtained by the further synchrotron radiation characterization and fitting where Ba and Sr exist individually as single atoms, there may be a situation that the spacing between Sr and Ba is 3.8-4.0 Å.

Through the above technical solutions, the present invention can at least achieve the following beneficial effects:
(1) The single-atom catalyst provided by the present invention can effectively improve the adjustability in the oxidative coupling reaction of methane through the synergism between the carrier and the supported active metal, and in the presence of at least two active metals, further through the synergism between the active metals.
(2) The preparation method of the single-atom catalyst provided by the present invention is simple and easy to implement, with readily available raw materials, is capable of achieving a large-scale production, and has a potential for the industrial application.
(3) When used in the oxidative coupling reaction of methane, the single-atom catalyst provided by the present invention can also increase the selectivity to the hydrocarbons having two or more carbon atoms while maintaining a low methane activation temperature, thereby reducing the energy consumption of the oxidative coupling reaction of methane and laying the foundation for the industrial application.

### Examples

The present invention will be described in detail below through the examples. It should be understood that the following examples are only used to further illustrate and explain the content of the present invention in an exemplary manner, and are not intended to limit the present invention.

In the following examples, unless otherwise specified, the reagents used are commercially available products purchased from the regular chemical suppliers, with a purity of analytical grade.

The structure of the single-atom sites in the catalyst is characterized by XAFS. For example, the K-edge X-ray absorption spectra of Ba and Sr are tested at the XAS beamline (BL₁4W₁) of the China Shanghai Synchrotron Radiation Facility (SSRF, 3.5 GeV, 250 mA). A Si(311) double crystal monochromator is used to monochromatize the hard X-rays. Pd foil and PdO are used as standard compounds for testing, and the energy calibration is carried out.

The method for testing the proportion of active metal in a single-atom state relative to the total amount of active metal in the single-atom catalyst is as follows: characterization is performed using a synchrotron radiation method; ten random positions of the sample to be tested are selected for testing, and the fitting results indicate the ratio of the number of the positions in a single-atom state to the total number of the positions. For example, if nine out of ten to-be-tested positions on the same sample show a single-atom form, it is considered that the active metal in a single-atom state accounts for 90% of the total amount of the active metal. Here, ten positions are only used to explain the calculation method, and in actual testing, more than ten positions can be used in practice.

The calculation method for the proportion of the single-atoms M1 and M2 having a spacing of 3.8-4.0 Å between M1 and M2 relative to the total single-atom-state active metal is as follows: based on the bond length differences of Ba-O-Ba/La and Ba-O-Sr, the proportion of Ba-O-Sr is obtained by EXAFS (Extended X-ray Absorption Fine Structure) fitting. The EXAFS fitting method is specifically as follows: the collected EXAFS data are processed according to the standard procedure by using Athena and Artemis implemented in the IFEFFIT information software package. The parts details are described as follows: the acquired EXAFS data are processed according to the standard procedure by using the Athena module implemented in the IFEFFIT software package. EXAFS spectra are obtained by subtracting the post-edge background from the total absorption and then normalizing relative to the edge jump step. Subsequently, the x(k) data are Fourier transformed to the R space by using a hanning window (dk=1.0 Å-1) to separate EXAFS contributions from different shell layers. To obtain quantitative structural parameters around the central atom, the least squares curve parameter fitting is performed by using the ARTEMIS module in the IFEFFIT software package. The second shell layer is obtained by fitting.

The following EXAFS equation is used: $χ \left(k\right) = {\sum }_{j} \frac{{N}_{j} {S}_{o}^{2} {F}_{j} \left(k\right)}{{kR}_{j}^{2}} exp \left[-2{k}^{2}{σ}_{j}^{2}\right] exp \left[\frac{- 2 {R}_{j}}{λ \left(k\right)}\right] sin \left[2{kR}_{j}+{ϕ}_{j}\left(k\right)\right]$ where So² is the amplitude reduction factor, Fⱼ(k) is the effective curved-wave backscattering amplitude, Nⱼ is the number of neighbors in the j-th atomic shell layer, Rⱼ is the distance between the X-ray absorbing central atom and the atom in the j-th atomic shell layer (backscatterers), λ is the mean free path in Å, φⱼ(k) is the phase shift (including the phase shift of each shell layer and the total phase shift of the central atom), and σⱼ is the Debye-Waller parameter for the j-th shell layer (variation in distance around the mean Rⱼ). The functions Fⱼ(k), λ, and ϕⱼ(k) are calculated by using the ab initio code FEFF8.2. The coordination numbers of the model samples are fixed to nominal values. The resulting So² is fixed in subsequent fittings. While the internal atomic distance R, Debye-Waller factor σ₂, and the edge energy shift ΔE₀ are allowed to proceed freely.

### Example 1

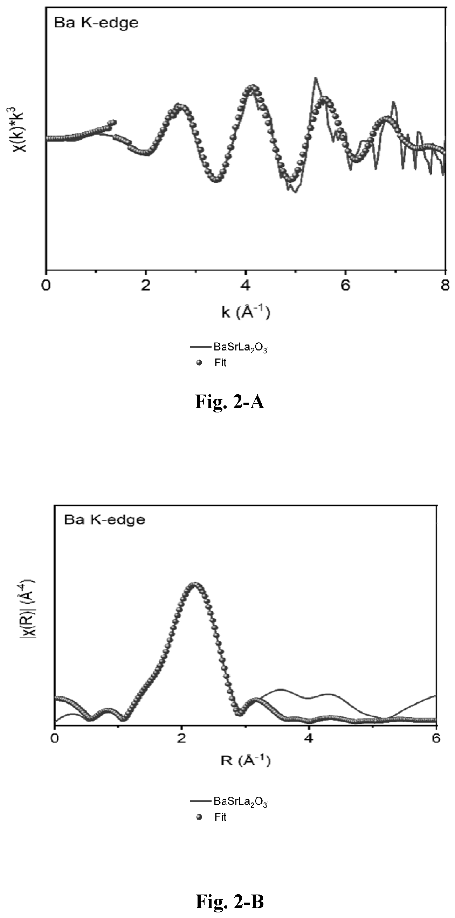

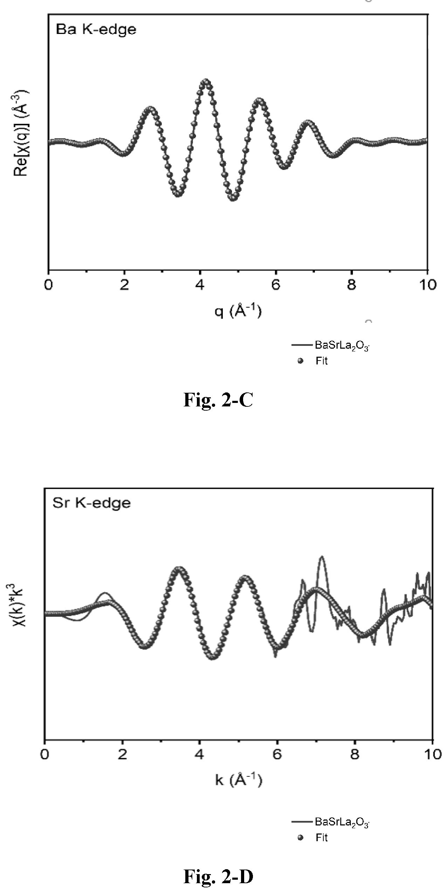

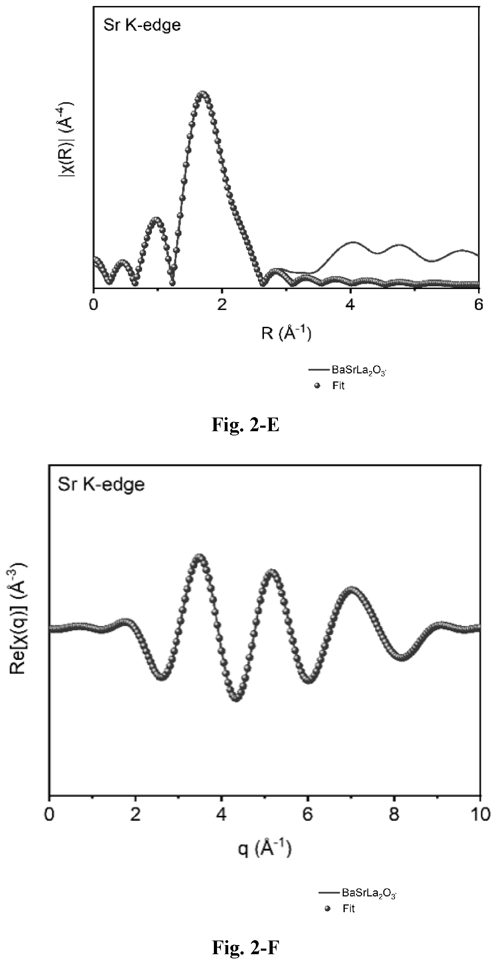

Preparation of the single-atom catalyst CAT-A containing the metals of barium and strontium using the following method:
Carrier pretreatment: Lanthanum oxide was placed at 350°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 20 mL of ethanol solution (95% concentration). Then, 1.8 g of strontium nitrate and 1.5 g of barium nitrate were weighed and dissolved in 25 mL of deionized water, and 5 mol% (calculated based on lanthanum oxide) of diethylenetriamine multidentate ligand was added to obtain a solution. This solution was added to the above lanthanum oxide ethanol solution. An ultrasonic dispersion was carried out with stirring at 50°C for 4 hours (ultrasonic frequency 40 kHz, ultrasonic power 300 W), followed by filtration and drying to obtain a catalyst precursor. The obtained catalyst precursor was dried in a drying oven at 80°C for 12 hours. Finally, it was calcined at 500°C for 2 hours and cooled to obtain the catalyst CAT-A. A synchrotron radiation was used for characterization (XAFS spectra). It showed that Ba mainly existed in the form of the Ba-O bond coordination, with a bond length of about 2.75 Å and a coordination number of 3.9; Sr also coordinated with O to form an Sr-O bond, with a bond length of about 1.97 Å and a coordination number of 2.9. Fitting the results (see Figures 2-A to 2-F) revealed that this catalyst CAT-A contained the single-atom sites of two active metals of Ba-O-Sr, and there was a situation that a spacing between Ba and Sr is 3.97 Å.

In the single-atom catalyst, the proportion of the active metals in a single-atom state relative to the total active metals was 92%.

The proportion of Ba and Sr in a single-atom state having a spacing of 3.97 Å between Ba and Sr relative to the total single-atom-state active metals was 40%.

### Example 2

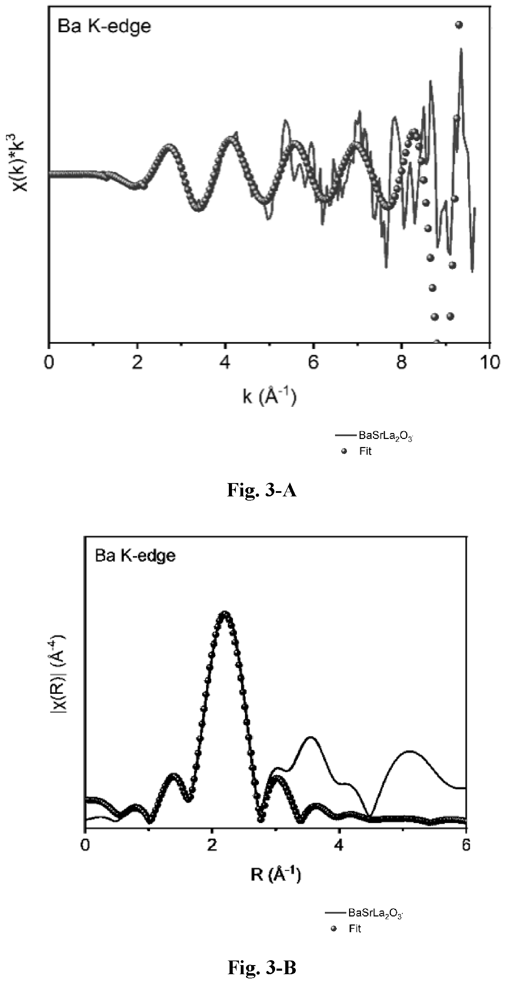

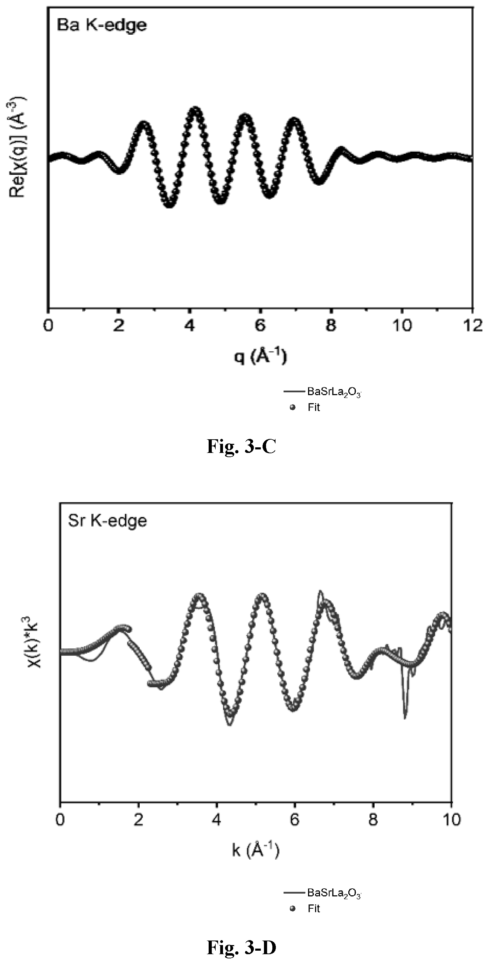

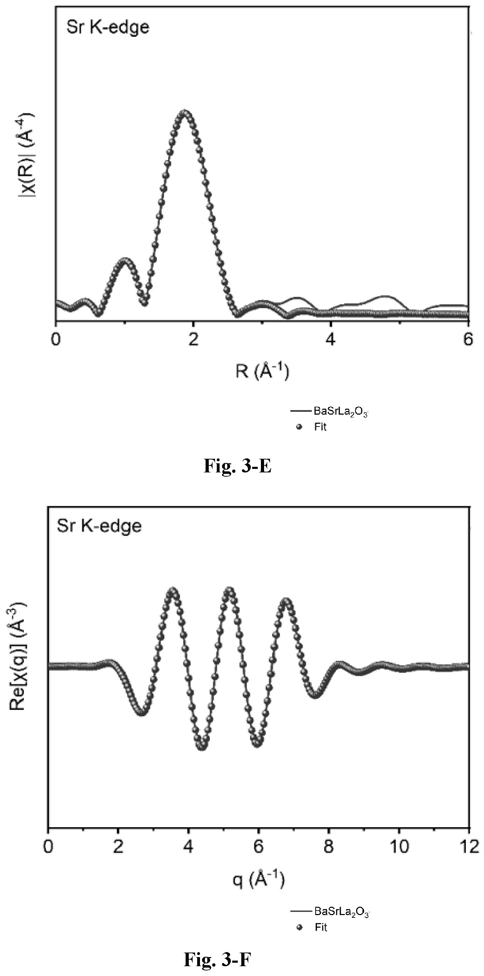

Preparation of the single-atom catalyst CAT-B containing the metals of barium and strontium using the following method:
Carrier pretreatment: Lanthanum oxide was placed at 350°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 25 mL of ethanol solution (95% concentration). Then, 0.9 g of strontium nitrate and 1.5 g of barium nitrate were weighed and dissolved in 25 mL of deionized water, and 5 mol% (based on lanthanum oxide) of ethylenediamine tetraacetic acid multidentate ligand was added to obtain a solution. This solution was added to the above lanthanum oxide ethanol solution. An ultrasonic dispersion was carried out with stirring at 60°C for 4 hours (ultrasonic frequency 50 kHz, ultrasonic power 200 W), followed by filtration and drying to obtain a catalyst precursor. The obtained catalyst precursor was dried in a drying oven at 80°C for 24 hours. Finally, it was calcined at 550°C for 2 hours and cooled to obtain the catalyst CAT-B. A synchrotron radiation was used for characterization (XAFS spectra). It showed that Ba mainly existed in the form of the Ba-O bond coordination, with a bond length of about 2.77 Å and a coordination number of 3.9; Sr also coordinated with O to form an Sr-O bond, with a bond length of about 1.97 Å and a coordination number of 3.0. Fitting the results (see Figures 3-A to 3-F) revealed that this catalyst CAT-B contained the single-atom sites of two active metals of Ba-O-Sr, and there was a situation that a spacing between Ba and Sr is 3.97 Å.

In the single-atom catalyst, the proportion of the active metals in a single-atom state relative to the total active metals was 95%.

The proportion of Ba and Sr in a single-atom state having a spacing of 3.97 Å between Ba and Sr relative to the total single-atom-state active metals was 28.8%.

### Example 3

Preparation of the single-atom catalyst CAT-C containing the metals of barium and strontium using the following method:
Carrier pretreatment: Lanthanum oxide was placed at 500°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 15 mL of ethanol solution (95% concentration). Then, 0.9 g of strontium nitrate and 1.5 g of barium nitrate were weighed and dissolved in 25 mL of deionized water, and 5 mol% (calculated based on lanthanum oxide) of bipyridine multidentate ligand was added to obtain a solution. This solution was added to the above lanthanum oxide ethanol solution. An ultrasonic dispersion was carried out with stirring at 60°C for 4 hours (ultrasonic frequency 80 kHz, ultrasonic power 400 W), followed by filtration and drying to obtain a catalyst precursor. The obtained catalyst precursor was dried in a drying oven at 80°C for 24 hours. Finally, it was calcined at 550°C for 2 hours and cooled to obtain the catalyst CAT-C. A synchrotron radiation was used for characterization (XAFS spectra). It showed that Ba mainly existed in the form of the Ba-O bond coordination, with a bond length of about 2.77 Å and a coordination number of 3.32; Sr also coordinated with O to form an Sr-O bond, with a bond length of about 1.97 Å and a coordination number of 2.54. Fitting the results revealed that this catalyst CAT-C contained the single-atom sites of two active metals of Ba-O-Sr, and there was a situation that a spacing between Ba and Sr is 3.83 Å.

In the single-atom catalyst, the proportion of the active metals in a single-atom state relative to the total active metals was 89%.

The proportion of Ba and Sr in a single-atom state having a spacing of 3.83 Å between Ba and Sr relative to the total single-atom-state active metals was 32%.

### Example 4

Preparation of the single-atom catalyst CAT-D containing the metals of barium and strontium using the following method:
Carrier pretreatment: Lanthanum oxide was placed at 450°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 20 mL of ethanol solution (95% concentration). Then, 1.8 g of strontium nitrate and 1.3 g of barium nitrate were weighed and dissolved in 25 mL of deionized water, and 5 mol% (calculated based on lanthanum oxide) of porphyrin multidentate ligand was added to obtain a solution. This solution was added to the above lanthanum oxide ethanol solution. An ultrasonic dispersion was carried out with stirring at 60°C for 4 hours (ultrasonic frequency 100 kHz, ultrasonic power 200 W), followed by filtration and drying to obtain a catalyst precursor. The obtained catalyst precursor was dried in a drying oven at 80°C for 24 hours. Finally, it was calcined at 550°C for 2 hours and cooled to obtain the catalyst CAT-D. A synchrotron radiation was used for characterization (XAFS spectra). It showed that Ba mainly existed in the form of the Ba-O bond coordination, with a bond length of about 2.75 Å and a coordination number of 3.11; Sr also coordinated with O to form an Sr-O bond, with a bond length of about 1.97 Å and a coordination number of 2.13. The R-space fitting of the results revealed that this catalyst CAT-D contained the single-atom sites of two active metals of Ba-O-Sr, and there was a situation that a spacing between Ba and Sr is 3.85 Å.

In the single-atom catalyst, the proportion of the active metals in a single-atom state relative to the total active metals was 85%.

The proportion of Ba and Sr in a single-atom state having a spacing of 3.85 Å between Ba and Sr relative to the total single-atom-state active metals was 35%.

### Example 5

Preparation of the single-atom catalyst CAT-E containing the metal of barium using the following method:
Carrier pretreatment: Lanthanum oxide was placed at 450°C and reduced with H₂ for 1 hour for performing the heat treatment A. The reduced lanthanum oxide had oxygen defect sites on the surface, and the specific surface area of the carrier was 58 m²/g.
Loading of the active metal and the ligand: 10 g of the reduced lanthanum oxide was weighed and ultrasonically dispersed in 20 mL of ethanol solution (95% concentration). Then, 1.3 g of barium nitrate was weighed and dissolved in 25 mL of deionized water, and 5 mol% (calculated based on lanthanum oxide) of porphyrin multidentate ligand was added to obtain a solution. This solution was added to the above lanthanum oxide ethanol solution. An ultrasonic dispersion was carried out with stirring at 60°C for 4 hours (ultrasonic frequency 100 kHz, ultrasonic power 200 W), followed by filtration and drying to obtain a catalyst precursor. The obtained catalyst precursor was dried in a drying oven at 80°C for 24 hours. Finally, it was calcined at 550°C for 2 hours and cooled to obtain the catalyst CAT-E. A synchrotron radiation was used for characterization (XAFS spectra). It showed that Ba mainly existed in the form of the Ba-O bond coordination, with a bond length of about 2.75 Å and a coordination number of 3.11. The R-space fitting of the results revealed that this catalyst CAT-E contained the single-atom sites of the active metal of Ba-O-Ba, and there was a situation that a spacing between Ba and Ba is 3.96 Å.

In the single-atom catalyst, the proportion of the active metal in a single-atom state relative to the total active metal was 90%.

The proportion of Ba in a single-atom state having a spacing of 3.96 Å between Ba and Ba relative to the total single-atom-state active metal was 25%.

### Comparative Example 1

This comparative example is used to compare the effect of using a different ligand during the preparation method on the prepared material structure.

Preparation of the single-atom catalyst CAT-F containing the metals of barium and strontium using the following method:
Carrier pretreatment: Lanthanum oxide was placed at 350°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 20 mL of ethanol solution (95% concentration).

Then, 1.8 g of strontium nitrate and 1.5 g of barium nitrate were weighed and dissolved in 25 mL of deionized water, and 5 mol% (calculated based on lanthanum oxide) of pyridine was added to obtain a solution. This solution was added to the above lanthanum oxide ethanol solution. An ultrasonic dispersion was carried out with stirring at 50°C for 4 hours (ultrasonic frequency 50 kHz, ultrasonic power 200 W), followed by filtration and drying to obtain a catalyst precursor. The obtained catalyst precursor was dried in a drying oven at 80°C for 12 hours. Finally, it was calcined at 500°C for 2 hours and cooled to obtain the catalyst CAT-F. A synchrotron radiation was used for characterization (XAFS spectra). It showed the presence of the Ba-O bond coordination, with a bond length of about 2.785 Å, and Sr also coordinated with O to form an Sr-O bond, with a bond length of about 1.95 Å. Fitting the results showed that a spacing of 3.8-4.0 Å between Ba and Sr was not detected.

In the single-atom catalyst, the proportion of the active metals in a single-atom state relative to the total active metals was 2%.

### Comparative Example 2

This comparative example is used to compare the effect of not using ultrasonic dispersion after adding the active metals during the preparation method on the material structure.

Preparation of the single-atom catalyst CAT-G containing the metals of barium and strontium using the following method:
Carrier pretreatment: Lanthanum oxide was placed at 350°C and reduced with H₂ for 1 hour for performing the heat treatment A. The reduced lanthanum oxide had oxygen defect sites on the surface, and the specific surface area of the carrier was 58 m²/g.
Loading of the active metals and the ligand: 10 g of the reduced lanthanum oxide was weighed and ultrasonically dispersed in 20 mL of ethanol solution (95% concentration). Then, 1.8 g of strontium nitrate and 1.5 g of barium nitrate were weighed and dissolved in 25 mL of deionized water, and 5 mol% (based on lanthanum oxide) of diethylenetriamine multidentate ligand was added to obtain a solution. This solution was added to the above lanthanum oxide ethanol solution. The mixture was stirred and dispersed with stirring at 50°C for 4 hours, followed by filtration and drying to obtain a catalyst precursor. The obtained catalyst precursor was dried in a drying oven at 80°C for 12 hours. Finally, it was calcined at 500°C for 2 hours and cooled to obtain the catalyst CAT-G. A synchrotron radiation was used for characterization (XAFS spectra). It showed Ba and Sr sites, the presence of the Ba-O bond coordination and a bond length of about 2.77 Å; Sr also coordinated with O to form an Sr-O bond, with a bond length of about 1.95 A. Fitting the results showed that a spacing of 3.8-4.0 Å between Ba and Sr was not detected.

In the single-atom catalyst, the proportion of the active metals in a single-atom state relative to the total active metals was 5%.

### Performance Test of the Catalysts from the Examples and the Comparative Examples

### Test Example 1

This test example is used to illustrate the catalytic performance of the catalysts obtained in the above examples in the oxidative coupling reaction of methane.

0.1 g of catalyst was loaded into a fixed-bed quartz reactor. Under the normal pressure condition, the molar ratio of methane to oxygen was 6:1, and the space velocity of methane was 40,000 mL/(g·h). The reaction activation temperature was measured, and the methane conversion and the selectivity to the hydrocarbons having two or more carbon atoms (C²⁺) were measured at the reaction temperatures of 500°C and 600°C. The results are shown in detail in Table 1. The activation temperature refers to the reaction temperature at which both the methane conversion is greater than 2% and the selectivity to the hydrocarbons having two or more carbon atoms is greater than 5%.

The specific detection method was as follows:
An Agilent 7890A gas chromatograph was used for the analysis of the components of the reaction products, and the methane conversion and the selectivity to the hydrocarbons having two or more carbon atoms (C²⁺) were calculated based on the analysis results. The yield of the hydrocarbons having two or more carbon atoms can be calculated by the product of the methane conversion and the selectivity to the hydrocarbons having two or more carbon atoms. ${CH}_{4} conversion = \frac{{C}_{{CH}_{4}}^{in} - {C}_{{CH}_{4}}^{out}}{{C}_{{CH}_{4}}^{in}} \times 100 %$ Formula (1)

In Formula (1), CH₄conversion refers to the methane conversion, ${C}_{{CH}^{4}}^{in}$ is the methane concentration at the reactor inlet, and ${C}_{{CH}^{4}}^{out}$ is the methane concentration at the reactor outlet. $C 2 + sel = \frac{2 {C}_{{C}_{2} {H}_{4}} + 2 {C}_{{C}_{2} {H}_{6}} + 3 {C}_{{C}_{3} {H}_{6}} + 3 {C}_{{C}_{3} {H}_{8}} + 4 {C}_{{C}_{4} {H}_{8}}}{2 {C}_{{C}_{2} {H}_{4}} + 2 {C}_{{C}_{2} {H}_{6}} + 3 {C}_{{C}_{3} {H}_{6}} + 3 {C}_{{C}_{3} {H}_{8}} + 4 {C}_{{C}_{4} {H}_{8}} + {C}_{{CO}_{2}} + {C}_{CO}}$ Formula (2)

In Formula (2), C2+sel (or C²⁺ selectivity) refers to the selectivity to the hydrocarbons having two or more carbon atoms, and C_{C2H4}, C_{C2H6}, C_{C3H6}, C_{C3H8}, C_{C4H8}, C_{CO2}, C_{CO} are the concentrations of C₂H₄, C₂H₆, C₃H₆, C₃H₈, C₄H₈, CO₂, CO at the reactor outlet respectively.

**Table 1**

| Catalyst | Activation Temperature/°C | 500°C | | 600°C | |
|---|---|---|---|---|---|
| | | CH₄conversion/% | C²⁺selectivity/% | CH₄conversion/% | C²⁺selectivity/% |
| CAT-A | 438 | 18 | 55.6 | 19.8 | 60.2 |
| CAT-B | 445 | 17.5 | 58.6 | 19.5 | 58.9 |
| CAT-C | 435 | 18.2 | 57.9 | 18.9 | 59.6 |
| CAT-D | 450 | 16.4 | 60.2 | 19.4 | 58.4 |
| CAT-E | 480 | 15.9 | 57.5 | 18.6 | 58.2 |
| CAT-F | 510 | 13.5 | 54.3 | 16.8 | 56.2 |
| CAT-G | 520 | 13.8 | 55.4 | 16.4 | 57.3 |

### Test Example 2

This test example is used to illustrate the change in the catalytic performance of the catalyst of the present invention under the different conditions of the oxidative coupling reaction of methane.

The catalytic performance of the catalyst CAT-A obtained from Example 1 was tested using the method in Test Example 1 according to the conditions in Table 2. The results are shown in detail in Table 2.

**Table 2**

| Reaction Conditions | | | Test Results | |
|---|---|---|---|---|
| Alkane/Oxygen ratio | Temperature (°C) | Space Velocity (mL·g⁻¹·h⁻¹) | CH₄conversion (%) | C²⁺selectivity (%) |
| 8:1 | 600 | 40000 | 19.6 | 59.6 |
| 10:1 | 600 | 40000 | 18.6 | 62.1 |
| 2:1 | 600 | 40000 | 32.3 | 41.2 |
| 1.5:1 | 600 | 40000 | 33.1 | 39.6 |
| 3:1 | 750 | 40000 | 30.4 | 43.2 |
| 3:1 | 800 | 40000 | 30.3 | 42.6 |
| 3:1 | 600 | 20000 | 29.6 | 44.5 |
| 3:1 | 600 | 160000 | 33.2 | 45.2 |
| 3:1 | 600 | 100000 | 30.1 | 43.5 |
| 3:1 | 600 | 10000 | 28.6 | 41.9 |
| 3:1 | 600 | 200000 | 33.6 | 45.1 |

| | | | | |
|---|---|---|---|---|
| Note: In Table 2, "Alkane/Oxygen ratio" refers to the molar ratio of methane to oxygen in the feed gas; the space velocity is the feed gas space velocity measured based on methane in the feed gas; the methane conversion and the selectivity of the hydrocarbons having two or more carbon atoms (C²⁺) were detected and calculated by using the method in Test Example 1. | | | | |

It was found by comparing the examples and the comparative examples that the technical solution of the present invention effectively improves the catalytic activity of the active metals in the catalyst, can significantly lower the reaction temperature of the oxidative coupling of methane, and make the industrial production of the oxidative coupling of methane possible.

Further stability tests were performed on the catalysts obtained in Examples 1-5. Under the conditions of Table 1 and Table 2, the catalysts were continuously reacted for 50 hours, and it was observed that the catalyst performance remained unchanged or essentially unchanged during the reaction.

The endpoints of the ranges and any values disclosed in the present invention are not limited to the precise ranges or values; these ranges or values should be understood to include values close to these ranges or values. For numerical ranges, between the endpoint values of each range, between the endpoint values and the individual point values of each range, and between the individual point values, they can be combined with each other to obtain one or more new numerical ranges, which should be considered as specifically disclosed in the present invention.

The preferred embodiments of the present invention are described in detail above, but the present invention is not limited thereto. Within the scope of the technical concept of the present invention, various simple modifications can be made to the technical solution of the present invention, including combining the various technical features in any other suitable manner. These simple modifications and combinations should also be regarded as the content disclosed by the present invention and fall within the protection scope of the present invention.

## Claims

1. A single-atom catalyst, which comprises a carrier and an active metal supported on the carrier, wherein the carrier is an oxide carrier, the active metal is selected from at least one of Group IA metals, Group IIA metals, Group IIB metals, Group IIIB metals, Group VIB metals and Group VIIB metals,
wherein the active metal present in a single-atom state on the carrier accounts for ≥70%, preferably ≥80%, more preferably ≥90% of the total amount of the active metal.

2. The single-atom catalyst according to claim 1, which has at least one of the following features:
based on a non-oxygen element in the carrier and a metal element in the active metal, a molar ratio of the carrier to the active metal is 2-500:1, preferably 4-500:1, more preferably 4-400:1;
in the single-atom catalyst, there is a situation that a spacing between the active metal atoms is 3.8-4.0 Å;
in the single-atom catalyst, there is an M1-(O)ₙ-M2 structural form, wherein M1 and M2 respectively represent the same active metal or two different active metals, n represents 1 or 2, preferably 1, and a spacing between M1 and M2 is 3.8-4.0 Å; and
the active metal is selected from at least one of Sr, Ba, Mn, Mg, Ce, Ca, Li, Na, K, Zn and W, preferably a combination of any two, particularly preferably Sr and Ba.

3. The single-atom catalyst according to claim 1 or 2, which has at least one of the following features:
the proportion of the single-atom-state metals having a spacing of 3.8-4.0 Å between the active metal atoms relative to the total single-atom-state active metal is ≥25%;
the proportion of the single-atom-state M1 and M2 having a spacing of 3.8-4.0 Å between M1 and M2 relative to the total single-atom-state active metal is ≥25%;
the carrier is selected from at least one of lanthanum oxide, aluminum oxide, cerium oxide and silicon oxide, preferably lanthanum oxide;
the carrier is in a crystalline or amorphous state; and
a specific surface area of the carrier is 1-500 m²/g, more preferably 20-400 m²/g.

4. The single-atom catalyst according to any one of claims 1-3, which has at least one of the following features:
at least two active metals, preferably two active metals, are present in the single-atom catalyst, and the active metals are a combination of at least two metals selected from the same Group, or a combination of at least two metals selected from different Groups;
the active metal is Ba and/or Sr;
the active metal is any one of the following combinations: Sr and Ba, Sr and Mn, Ba and Mg, Ce and Mg, Ba and Li, Ca and K, Ba and Mn, Zn and W, or Mn and Na; and
in the single-atom catalyst M1M2/S containing two active metals, based on metal elements, the molar ratio of M1 to M2 is 1:0.1-10, preferably 1:0.1-5.

5. A method for preparing the single-atom catalyst according to any one of claims 1-4, comprising:
Step 1: preparing a mixed solution from a precursor of at least one metal, a nitrogen-containing compound ligand containing two or more nitrogen atoms, and a solvent, and contacting the mixed solution with a carrier optionally subjected to a reduction treatment, to obtain a mixture;
Step 2: subjecting the mixture obtained in Step 1 to an ultrasonic dispersion, followed by filtration and drying, to obtain a catalyst precursor;
Step 3: drying and calcining the catalyst precursor obtained in Step 2, and cooling to obtain a single-atom catalyst;
wherein the carrier is an oxide carrier, and the metal is selected from at least one of Group IA metals, Group IIA metals, Group IIB metals, Group IIIB metals, Group VIB metals and Group VIIB metals.

6. The preparation method according to claim 5, which has at least one of the following features:
the carrier is selected from at least one of lanthanum oxide, aluminum oxide, cerium oxide and silicon oxide, preferably lanthanum oxide;
the metal is selected from at least one of Sr, Ba, Mn, Mg, Ce, Ca, Li, Na, K, Zn and W, preferably a combination of any two, particularly preferably Sr and Ba;
when at least two active metals, preferably two active metals, are used, the metals are a combination of at least two metals selected from the same Group, or a combination of at least two metals selected from different Groups;
the metal is Ba and/or Sr; and
the metal may be any one of the following combinations: Sr and Ba, Sr and Mn, Ba and Mg, Ce and Mg, Ba and Li, Ca and K, Ba and Mn, Zn and W, or Mn and Na.

7. The preparation method according to claim 5 or 6, which has at least one of the following features:
the precursor is a salt, which is preferably selected from water-soluble salts, more preferably at least one of nitrates, sulfates and halides, wherein said halides are, for example, fluorides, chlorides and bromides;
the nitrogen-containing compound ligand is selected from (organic) compounds containing an amine group;
the nitrogen-containing compound ligand is selected from at least one of diethylenetriamine, ethylenediamine tetraacetic acid, bipyridine, porphyrin and phthalocyanine;
the solvent is water or an organic solvent, more preferably, the solvent is at least one of water, alcohols having no more than 5 carbon atoms, and ketones having no more than 5 carbon atoms, even more preferably, the solvent is at least one of water, methanol, ethanol and acetone; and
in the mixed solution, the molar concentration ratio of the ligand to the precursor, calculated based on metal ions, is 2-5:1.

8. The preparation method according to any one of claims 5-7, which has at least one of the following features:
the carrier is pretreated before contacting with the mixed solution, preferably the pretreatment method may be a heat treatment A performed on the carrier under a reducing atmosphere;
calculated based on metal ions, the amount of the carrier as used is such that the molar ratio of the carrier to the metal precursor is 2-500:1, preferably 4-500:1, more preferably 4-400:1;
in Step 2, after the ultrasonic treatment, a heat treatment B is further performed;
the conditions of the ultrasonic treatment include: a temperature of 40-70°C, an ultrasonic frequency of 20-100 kHz, an ultrasonic power of 200-500 W, an ultrasonic time 0.5-5 h;
stirring is performed during the ultrasonic treatment;
the drying conditions are such that the solvent content in the carrier loaded with the metal precursor and the ligand does not exceed 1% by weight, preferably 0.1-0.7% by weight;
the calcination method includes: calcining at 100-650°C for 30-300 minutes; and
the calcination is carried out under a protective atmosphere.

9. The preparation method according to any one of claims 5-8, which has at least one of the following features:
the reducing atmosphere is provided by H₂ and/or CO;
the conditions of the heat treatment A include: a temperature of 300-500°C, time of 0.5-5 h, for example, the carrier can be left to stand under the above conditions;
the conditions of the heat treatment B include: a temperature of 40-90°C, time of 1-10 h; and
the conditions of the drying include: a temperature of 70-120°C, time of 10-30 hours.

10. A use of the single-atom catalyst according to any one of claims 1-4 or the single-atom catalyst obtained by the preparation method according to any one of claims 5-9 in an oxidative coupling reaction of methane.

11. A use of the preparation method according to any one of claims 5-9 in an oxidative coupling reaction of methane.

12. A method for producing hydrocarbons having two or more carbon atoms, which comprises contacting a feed gas comprising CH₄ and O₂ with the single-atom catalyst according to any one of claims 1-4 or the single-atom catalyst obtained by the preparation method according to any one of claims 5-9, to carry out an oxidative coupling reaction of methane.

13. The method according to claim 12, wherein
the molar ratio of CH₄ to O₂ in the feed gas is 2-10:1, preferably 2-8:1; and/or
the conditions for the oxidative coupling reaction of methane include: a reaction temperature of not lower than 450°C, preferably 480-800°C; a feed gas space velocity, based on CH₄, of 20,000-200,000 mL/(g·h), preferably 30,000-180,000 mL/(g·h).
